# EUROPEAN PATENT APPLICATION

(11) **EP 1 300 472 A1**
(43) Date of publication of application: **09.04.2003**
(21) Application number: 01203745.3
(22) Date of filing: 02.10.2001
(51) Int. Cl.: C12Q 1/10, G01N 33/569

(54) **A method for testing the effect of nutrients on gastrointestinal colonisation resistance of humans**

(71) Applicant: Stichting Top-Instituut Voedselwetenschappen, 6700 AN Wageningen (NL)
(72) Inventor: Bodee-Oudenhoven, Ingeborg Marie Jacqueline, 6604 GL Wijchen (NL); Van der Meer, Roelof, 6712 HA Ede (NL)
(74) Representative: Jorritsma, Ruurd

(57) **Abstract**

The present invention relates to a method for testing the effect of a substance, in particular a food-ingredient, on the resistance to microbial infection of the human gastrointestinal tract. The microorganism may be a gastrointestinal pathogen, such as a virus, a bacterium, a fungus or a protozoan organism, in which case usually a attenuated or non-virulent strain of the microorganism, such as a live oral vaccine, will be applied in the method . Alternatively, the method may be applied to test the effect of the substance on the capability of non-pathogenic beneficial bacterium to colonise the intestinal mucosa. In a specific example, the method demonstrates that calcium reduces the severity of infection by an enterotoxigenic *E.coli* as well as the clinical symptoms associated with the infection. In a further aspect the invention therefore relates to a method for preventing or reducing the severity of a gastrointestinal infection by a Gram-negative pathogenic bacterium by increasing the gastrointestinal calcium concentration.

## Description

### Field of the invention

The present invention relates to a method for testing in humans the effect of nutrients on the colonisation or infection by gastrointestinal microorganisms.

### Background of the invention

Food-borne infections or gastrointestinal infections are highly prevalent in man. In the Netherlands on average 10% of the population suffers from a food-borne infection at least once in a year. For travellers to tropical regions this percentage can even reach up to 80%. Usually, after a few days of diarrhoea, abdominal cramps, nausea, fever and/or vomiting the patient recovers from the infection. However, in people with reduced resistance, such as new-borns, the elderly and immunocompromised subjects, gastrointestinal infections may lead to live-threatening complications. Treatment of food-borne infections with antibiotics is often not very effective. Moreover, bacterial pathogens are increasingly becoming resistant to these compounds.

For these reasons it is important that alternative methods for the prevention or treatment of gastrointestinal infections are developed. In addition to improved hygiene, one alternative approach is to adapt the diet to increase the resistance of the human subjects to food-borne infections. The composition of the ingested food largely determines the content of the gastrointestinal tract, which, in turn, affects (1) the survival of bacterial pathogens during its passage through the gastrointestinal tract, (2) the beneficial and protective microflora, and (3) the barrier function of the intestinal mucosa. These three aspects together determine for the most part the primary defence against food-borne infections.

Various animal studies have shown that bacterial colonisation of the gut depends on the composition of the diet, and in particular on its calcium concentration (Bovee-Oudenhoven et al., 1997 Gastroenterol. 113: 550-557; Bovee-Oudenhoven et al., 1999 J. Nutr. 129: 607-612). Dietary calcium inhibits both the colonisation (attachment and bacterial multiplication), as well as, the invasion (penetration of the intestinal mucosa and infection of other organs) by *Salmonella.* Probably, the calcium-induced increase in resistance against infection is the result of several factors working together to combat the infection. Each of stimulation of the lactobacilli in the intestinal microflora, reduction of damage to the intestinal mucosa by inactivation of irritating compounds in the intestinal content, as well as, direct binding of calcium salts to bacterial pathogens could be responsible for the beneficial effect of calcium. It is not yet known whether calcium is also beneficial with respect to food-borne infections in man.

Thus, there is a need for a method for testing the effects of calcium and other nutrients on the resistance of humans against food-borne infections. This need is particularly important in view of the trend among the regulatory authorities and the public to demand substantiation of health claims made for a particular food-products or -ingredients.

### Description of the invention

In a first aspect the present invention relates to a method for testing the effect of a dietary substance on the resistance to microbial infection of the human gastrointestinal tract. The method comprises the steps of (a) acquiring at least two of human volunteers; (b) separating the volunteers into two or more groups; (c) putting each group on a different diet whereby the diets differ at least in the amount of the substance present in the diet; (d) oral administration of a viable microorganism to the volunteers, while the volunteers are on the diet; (e) determining on the volunteers a clinical parameter indicative of the presence of the microorganism in the gastrointestinal tract while the volunteers are on the diet (f) comparing the clinical parameter indicative of the presence of the microorganism in the gastrointestinal tract, between the different groups to determine the effect of the substance on the colonisation or infection of the human gastrointestinal tract by the microorganism.

The effect of the substance on the colonisation or infection by the microorganism is preferably determined by measuring the effect of the substance on a clinical parameter associated with the administration of the microorganism, or rather, a parameter indicative of the presence of the microorganism in the gastrointestinal tract. Thus a given substance may reduce or increase the clinical parameter or the substance may not affect the clinical symptoms. Alternatively, the substance may influence the duration of the deviation of the clinical parameter indicative of the presence of the microorganism in the gastrointestinal tract as compared to the normal value of the parameter in subjects in which the microorganism is not present. The clinical parameter may be a clinical symptom associated with infection or colonisation by the microorganism, such as e.g. a gastrointestinal pathogen. Such clinical symptoms may e.g. include diarrhoea, nausea, headache, abdominal pain or cramps, fever, vomiting and perspiration. Further clinical parameters that may be used to determine the effect of the substance on the colonisation or infection by the microorganism include e.g. an antibody response against the microorganism's antigens.

A preferred clinical parameter to be determined is the colonisation resistance to the microorganism. The colonisation resistance to a microorganism, usually a bacterium, is inversely proportional to the excretion of the microorganism in faeces over time. Thus, if a substance in a diet has a positive effect on the colonisation resistance to a given pathogen, the pathogen disappears more rapidly from (or is present in lower amounts in) faecal excretions of subjects on a diet comprising the substance, as compared to subjects on a diet without the substance or with lower amounts of the substance. The colonisation resistance against pathogens therefore preferably is as high as possible. In contrast, the colonisation resistance against e.g. non-pathogenic beneficial bacteria (probiotics) preferably is as low as possible. A preferred method of the invention, therefore is a method for testing the effect of a substance on the colonisation resistance of a microorganism in humans, wherein the method comprises the steps of: (a) acquiring at least two of human volunteers; (b) separating the volunteers into two or more groups; (c) putting each group on a different diet whereby the diets differ at least in the amount of the substance present in the diet; (d) oral administration of the microorganism to the volunteers, while the volunteers are on the diet; (e) determining the amount of the microorganism in a faecal sample obtained from the volunteers while the volunteers are on the diet; and, (f) comparing the amounts of the microorganism in the faecal samples between the different groups to determine the effect of the substance on the colonisation resistance to the microorganism.

The volunteers are preferably included on the basis of informed consent and preferably are healthy. The volunteers may either be male or female although per individual study, preferably volunteers of one and the same sex are included. The volunteers preferably have a minimum age of 20 or 21 years and preferably a maximum age of 50, 55 or 60 years. In a particular embodiment of the invention the volunteers are included on the basis of a more narrow range of ages, e.g. differing no more than 5, 10, 15 or 20 years in age.

Preferred criteria for exclusion of volunteers are:
- a history of illnesses of or surgery on the gastrointestinal tract.
- lactose intolerance
- use of one or more of: antibiotics, activated charcoal (Norit™), laxatives (including e.g. lactulose preparations), cholestyramine, antacids, immunosuppressants during the last 3, 6, or 12 months prior to the study.
- serum antibody titres against the microorganism to be applied in the method of the invention.
- carrier of microorganisms resistant to a drug, such as an antibiotic, that is used as marker for the microorganism to be applied in the method of the invention.

The volunteers may further be asked to keep a diary in order to verify that the diets are faithfully followed.

The numbers of volunteers to be included per group is determined by power analysis. For that purpose the smallest difference of clinical relevance and the usual amount of variability (of continuous variables) needs to be specified. Power analysis calculates the sample size necessary to have a high probability of obtaining a statistically significant result if that is a true difference. Methods for power analysis are known per se to the skilled person (see e.g. Dawson-Saunders B and Trapp RG. Basic & clinical biostatistics, pp 275-276; 1994 sec edition; Appleton & Lange, Norwalk, Connecticut).

The substance to be tested preferably is a substance suitable for human consumption. The substance may be a compound or may be a composition comprising one or more compounds. Preferably the substance is or comprises a nutrient or a food-additive such as a carbohydrate, a protein, a lipid, a mineral a vitamin, an antioxidant, a preserving agent, an emulsifying agent, a food acid, a prebiotic, a nutraceutical or one or more (potentially) probiotic bacterial strains, and the like. Likewise, the substance may also be a complete food product, comprising one or more of the aforementioned substances.

In the method of the invention, each group of volunteers is put on a different diet whereby the diets differ at least in the amount of the substance present in the diet. Preferably, the amount of the substance in the diet of the group put on the diet with the highest amount of the substance differs at least by a factor 2, 5, 10, 50, 100, 1000 with the amount of the substance in the diet of the group put on the diet with the lowest amount of the substance. Particularly in the case substance is a probiotic, the amount in the respective diets may at least differ by a factor 10⁴, 10⁶, 10⁸, or 10¹⁰. The amount of the substance in the diet of the group put on the diet with the lowest amount of the substance may be below the detection limit or the diet may be completely void of the substance. The volunteers are preferably put on the diets at least 1, 2, 4, 7, 15 days or 1 month prior to the administration of the microorganism. Likewise, the volunteers are preferably kept on the diets at least 1, 2, 4, 7, 15 days or 1 month subsequent to the administration of the microorganism. The lengths of time of the diets prior to and after the administration of the microorganism will depend on both the substance and the microorganism to be tested. Some indication of the optimal length of time may be obtained in e.g. a pilot experiment using a limited number of volunteers or, although less reliable, in an animal model.

The microorganism to be tested in the method of the invention may be a virus, a bacterium, a fungus, or a protozoan microorganism. The microorganism may be a cellular or unicellular microorganism. Preferably, the microorganism is a pathogen capable of infecting the human gastrointestinal tract. In such instances preferably a non-virulent, attenuated and/or non-pathogenic strain of the pathogen is administered in the method in order to at least avoid health risks to the volunteers and preferably also to avoid inconvenience, i.e. preferably the strain to be administered in the method will only cause mild or no clinical symptoms in the volunteers. Such attenuated, non-virulent and/or non-pathogenic strains of these pathogenic microorganism are often available as live oral vaccines. The microorganism preferably carries a marker to facilitate its detection in faeces. Preferably the marker allows to isolate or identify the microorganism directly from faeces. Suitable markers include antibiotic resistance, the capability to grow on a particular carbon- and/or nitrogen compound as sole carbon- and/or nitrogen source, auxothropic markers or screenable markers such as Green Fluorescent Protein, Luciferase or β-galactosidase (lacZ).

In a preferred method of the invention, the microorganism is a bacterium. The bacterium preferably is a bacterium capable of colonising the human intestine, at least temporarily. The bacterium may be a pathogenic or a non-pathogenic bacterium and may be a Gram-positive or Gram-negative bacterium. The non-pathogenic bacterium to be applied in the method of the invention preferably is a non-pathogenic beneficial bacterium capable of colonising the intestinal mucosa, preferably with health promoting properties, a so-called probiotic.

In a preferred embodiment of the invention an enterotoxigenic *Escherichia coli* (ETEC) is used. Preferably, a variant ETEC strain is used that does not produce enterotoxin and that may be used as live oral vaccine against pathogenic ETECs, such as e.g. ETEC strain E1392-75-2A as described in Tacket and Levine, (1997), in: "New generation vaccines", Eds. M.M. Levine, G.C. Woodrow, J.B. Kaper and G.S Cobon, Marcel Dekker, Inc. New York. ETEC strain E1392-75-2A was deposited on September 27, 2001 and assigned accession number CBS 109740. Other live oral vaccines that may be used in the method of the invention are reviewed by M.M. Levine (1990) The Lancet, Apr 21, 335 (8695): 958-961. Further examples of gastrointestinal pathogenic bacteria which may be applied in the method of the invention include species belonging to the genus, *Bacillus, Bacteroides, , Campylobacter, Clostridium, Escherichia coli, Klebsiella, Listeria, Proteus, Pseudomonas, Salmonella, Shigella, Staphylococcus, Streptococcus, Vibrio, and Yersinia.* Again, these pathogenic bacteria are preferably used in attenuated, non-virulent or non-pathogenic form and are often available as live oral vaccines.

Examples of non-pathogenic Gram-positive bacteria that may be applied in the method of the invention include e.g. bacteria that belong to a genus selected from the group consisting of *Lactobacillus, Lactococcus, Leuconostoc, Streptococcus, Bifidobacterium, Eubacterium, Propionibacterium, Enterococcus, Staphylococcus,* and *Peptostreptococcus* A further preferred bacterium to be applied in the method of the invention is a *Lactobacillus* or *Bifidobacterium* species selected from the group consisting of *L. reuteri, L. fermentum, L. acidophilus, L. crispatus, L. gasseri, L. johnsonii, L. plantarum, L. paracasei, L. murinus, L. jensenii, L. salivarius, L. minutis, L. brevis, L. gallinarum, L. amylovorus, B. bifidum, B. longum, B. infantis, B. breve, B. adolescente, B. animalis, B. gallinarum, B. magnum,* and *B. thermophilum.*

The microorganism may also be an intestinal virus, such as e.g. Norwalk virus or rotavirus, a (pathogenic) fungus capable of infecting the gastrointestinal tract, such as e.g. *Candida* species, or one of the (gastro)intestinal protozoa, such as e.g. *Cryptosporidium, Trichomonads, Histamonas, Spironucleus, Entamoeba, Coccidia, Toxoplasma, Sarcocystis* and *Giardia* (*Giardia lambia*).

In the method of the invention a single microbial strain may be tested. Alternatively, two or more different microbial strains or species may be tested simultaneously.

In the method of the invention the bacterium is preferably orally administered. Depending on the strain and its infective dose, preferably at least 10⁶, 10⁸, or 10¹⁰ CFU (Colony Forming Units) or viable cells, per microbial strain or species are administered. In case the microorganism is a virus preferably at least 10⁶, 10⁸, or 10¹⁰ PFU (Plaque Forming Units) per viral strain or species are administered. The microorganism may be administered in any form suitable for human consumption Preferably the microorganism is administered in a drinkable liquid, preferably the liquid has a pleasant flavour that masks any disagreeable taste of the microorganism or the medium it was grown in, such as e.g. fruit juice, or (flavoured) milk or other dairy products. Alternatively the microorganisms may be administered in a solid or semisolid form, such as e.g. mixed in a food product or in a capsule with e.g. an enteric coating. In the preparation of any of these formulations for oral administration (or consumption) the microorganism may be supplied in the liquid form, e.g. a suspension, as well as in a solid form, e.g. as freeze-dried microorganisms.

Subsequent to the administration of the microorganism, the quantity of the microorganism excreted in the faeces is determined in faecal samples obtained from the volunteers at least once and preferably while the volunteers are still on the diet. Preferably, the amount of the excreted microorganism is followed over time by collecting faecal samples from the volunteers at various time points subsequent to the administration of the microorganism and determining the quantity of the microorganism in the samples. Preferably, samples are collected at regular intervals of preferably 1, 2, 3, or 7 days. Preferably also prior to the administration of the microorganism faecal samples are collected from the volunteers in order to establish the absence of the microorganism to be tested.

Quantification of the microorganism in the faecal samples may be performed by a number of methods known in the art per se. Usually the microorganism is quantified by plating several serial dilutions of the faecal samples on appropriate solid (agar) media. Preferably the medium allows for direct selection of the microorganism. These methods allow to determine the concentration of the microorganism in a faecal sample. Preferably also the total amount of excreted faeces is determined such that the total amount of excreted microorganism may be calculated. The results of the quantification may be subjected to a statistical analysis, preferably using a software for statistical analysis such as e.g. Statistica™ (1999, StatSoft Inc. Tulsa, OK, USA).

Finally, in the method of the invention the amounts of the microorganism in the faecal samples are compared between the different groups to determine the effect of the substance on the colonisation resistance against the microorganism. The substance may thus increase, decrease or not affect the colonisation resistance to the microorganism tested.

In addition to the quantification of the microorganism in the faecal samples a number of other parameters of the volunteers may be determined during the course of the study. Faecal samples may further be analysed for *Lactobacilli*; *Enterobacteriaceae*; cations such as sodium, potassium, ammonium as a measure for % wet weight; mucine; soluble bile acids; soluble fatty acids; and cytotoxicity of faecal water, inflammatory markers (like myeloperoxidase). Urine samples may be analysed for calcium. Blood samples may be analysed for IgM, IgG and IgA against the microorganism tested and/or saliva samples may be analysed for sIgA against the microorganism tested.

In a further aspect the invention relates to a method for preventing or reducing the severity of a gastrointestinal infection by a Gram-negative pathogenic bacterium, wherein the method comprises increasing the gastrointestinal calcium concentration. The method preferably prevents or reduces the severity of clinical symptoms caused by the infection by the Gram-negative bacterium. Such clinical symptoms e.g. include diarrhea, nausea, headache, abdominal pain, fever, vomiting, perspiration, an antibody response against the bacterium's antigens and colonisation resistance to the bacterium. Preferably the gastrointestinal calcium concentration is increased by an increased enteral or oral intake of calcium. The calcium is preferably administered in a therapeutically effective amount, i.e. an amount that is effective in preventing or reducing the infection. Preferably the subject in which the infection is to be prevented or in which the severity of the infection is to be reduced is put on a diet with an increased calcium content, whereby the diet contains at least 200, 400, 800, 1200, 1600, or 2000 mg of calcium per day. When the method is applied to treat an infected person, i.e. to reduce the severity of the infection, the increased calcium is taken for at least 2 - 7 days. When the method is applied to prevent an infection, the increased calcium is preferably taken during the entire period that a person is at risk of being infected.

The increased gastrointestinal calcium is achieved by enteral or oral administration of a supply of calcium, i.e. a composition that is capable of releasing Ca²⁺ ions upon administration. The supply of calcium may also be incorporated into a diet. Preferably, the supply of calcium is suitable for human consumption, i.e. is physiologically acceptable, which will usually mean that it is not toxic or otherwise harmful to human or animals when administered orally. Preferably the supply of calcium is a calcium salt, preferably a soluble calcium salt, or a calcium salt soluble in the acidic stomach. Examples of preferred calcium salts include e.g. calcium carbonates, calcium acetate, calcium chloride, or calcium phosphates.

In yet another aspect the invention relates to the use of calcium in the manufacture of a medicament for use in any of the above methods of treatment to prevent or reduce the severity of a gastrointestinal infection by a Gram-negative pathogenic bacterium.

### Description of the figures

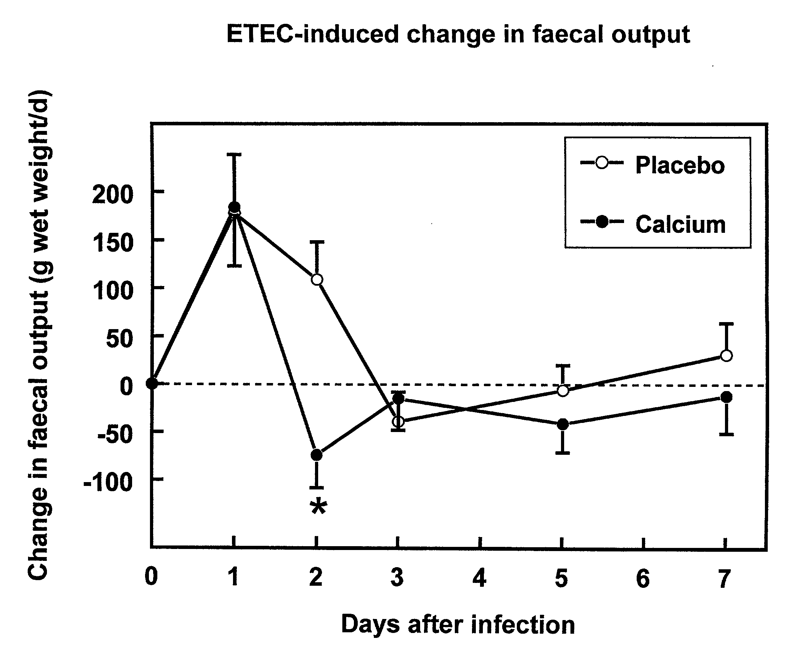

**Figure 1.** Change in faecal output upon administration of the ETEC vaccine in volunteers on the placebo diet as compared to volunteers on the high calcium diet. The change in faecal output is expressed in gram wet weight per day. The star (*) indicates the significant reduction in faecal output in the volunteers on the calcium diet on the second day after administration of the ETEC vaccine.

### Examples

### Example 1

The effect of dietary calcium on the resistance to enterotoxigenic Escherichia coli (ETEC) infection was determined in a parallel, placebo-controlled, double-blinded, human intervention study. During the experimental period of 3 weeks, healthy male adults (n=16 per group) consumed either regular milk and vanilla custard (calcium intake due to these milk products 1200 mg) or placebo milk and vanilla custard (calcium intake due to placebo milk products 120 mg). Volunteers were instructed to maintain their habitual diet, except that high-calcium foods and drinks (e.g. milk products other than the delivered ones, some vegetables, particular cookies) were forbidden. After an adaptation period of 10 days, the volunteers were vaccinated by the oral administration of a live ETEC vaccine (strain E1392-75-2A, dose 10¹⁰ colony-forming units). Before and after the vaccination the volunteers kept a diary to record their intake of foods and drinks (3x24 h) and possible discomfort due to the vaccination. In addition, the following biological materials were collected: blood, saliva, faeces, urine. Faecal samples collected after vaccination were analysed for ETEC excretion to determine intestinal ETEC colonisation. The ETEC strain used was streptomycin-resistant and could be cultured selectively. Additionally, faecal samples were analysed for several other infection-related parameters (e.g. relative faecal dry weight, mucins). Specific immune reponses to the ETEC strain administered were determined in blood and saliva. Both faeces and urine were analysed for calcium to check compliance of the volunteers to the dietary protocol.

The results of the ETEC excretion in faecal samples are shown in Table 1. Figure 1 shows the significant reduction in faecal output (gram wet weight per day) in volunteers on the calcium diet as compared to volunteers on the placebo diet.

**Table 1.**

| Effect of dietary calcium on fecal ETEC excretion 2 days after vaccination. | |
|---|---|
| **Group** | ***ETEC (log***_{***10***}**/*d)*** |
| Placebo | 11.36 ± 0.18 |
| Calcium | 10.61 ± 0.34 |

## Claims

1. A method for testing the effect of a substance on a microorganism's capability to colonise or infect the human gastrointestinal tract, wherein the method comprises the steps of:
(a) acquiring at least two of human volunteers;
(b) separating the volunteers into two or more groups;
(c) putting each group on a different diet whereby the diets differ at least in the amount of the substance present in the diet;
(d) enteral administration of a viable microorganism to the volunteers, while the volunteers are on the diet;
(e) determining on the volunteers a clinical parameter indicative of the presence of the microorganism in the gastrointestinal tract while the volunteers are on the diet; and,
(f) comparing the clinical parameter parameter indicative of the presence of the microorganism in the gastrointestinal tract, between the different groups to determine the effect of the substance on the colonisation or infection of the human gastrointestinal tract by the microorganism.

2. A method according to claim 1, wherein the clinical parameter is the colonisation resistance to the microorganism, and wherein the colonisation resistance is determined in step (e) by measuring the amount of the bacterium in a faecal sample obtained from the volunteers while the volunteers are on the diet; and, wherein the effect of the substance on the colonisation resistance is determined in step (f) by comparing the amounts of the bacterium in the faeces samples between the different groups.

3. A method according to claims 1 or 2, wherein the microorganism is an attenuated or non-virulent strain of a pathogenic microorganism capable of colonising the intestinal mucosa.

4. The method of claim 3, wherein the attenuated or non-virulent strain of a pathogenic microorganism is a live oral vaccine.

5. The method of claim 4, wherein the live oral vaccine is a variant of an enterotoxigenic *E. coli* that does not produce enterotoxins.

6. A method according to claim 1 or 2, wherein the microorganism is a non-pathogenic beneficial bacteria capable of colonising the intestinal mucosa.

7. A method according to any one of the preceding claims, wherein the amount of the substance in the diet of the group put on the diet with the highest amount of the substance differs at least by a factor 2 with the amount of the substance in the diet of the group put on the diet with the lowest amount of the substance.

8. A method according to any one of the preceding claims, wherein the substance is a nutrient or a food-additive selected from the group consisting of a carbohydrate, a protein, a lipid, a mineral a vitamin, an antioxidant, a preserving agent, an emulsifying agent, a food acid, a prebiotic, a nutraceutical and probiotic bacterium.

9. A method according to any one of claims 1 - 7, wherein the substance is a food product.

10. A method according to any one of the preceding claims, wherein the volunteers are put on the diets at least 7 days before the administration of the bacterium, and wherein the volunteers are kept on the diets at least 7 days after the administration of the bacterium, and whereby the amount of the bacterium in faecal samples is determined at least twice after the administration of the bacterium.

11. A method for preventing or reducing the severity of a gastrointestinal infection by a Gram-negative pathogenic bacterium, wherein the method comprises increasing the gastrointestinal calcium concentration.

12. Use of calcium in the manufacture of a medicament for use in a method of treament to prevent or reduce the severity of a gastrointestinal infection by a Gram-negative pathogenic bacterium.
